# EUROPEAN PATENT SPECIFICATION

(11) **EP 0 639 359 B1**
(45) Date of publication and mention of the grant of the patent: **17.03.1999**
(21) Application number: 94107817.2
(22) Date of filing: 20.05.1994
(51) Int. Cl.: A61F 2/40, A61F 2/30

(54) **An endoprothesis for a shoulder joint**
Endoprothese für Schultergelenk
Endoprothèse pour l'épaule

(30) Priority: 16.08.1993 DE 9312218 U
(43) Date of publication of application: 22.02.1995
(73) Proprietor: Howmedica GmbH, D-24232 Schönkirchen (DE)
(72) Inventor: Resch, Herbert F., A-6080 Innsbruck (AT); Müller, Holger, D-24214 Gettorf (DE); Robioneck, Bernd, D-24211 Schellhorn (DE)
(74) Representative: Patentanwälte Hauck, Graalfs, Wehnert, Döring, Siemons

(56) References cited:
- FR-A- 2 349 319
- FR-A- 2 578 739
- US-A- 3 102 536

## Description

The present invention relates to an endoprosthesis for a shoulder joint as defined by claim 1.

An endoprosthesis of this type usually includes a tapered plug-in connection between a head portion including a spherical joint surface and the elongate shank portion. The axis of the plug-in connection coincides with the axis of the shank and the center of curvature of the joint surface is spaced from said axis.

For this structure the distance between the joint surface or, respectively, the center of curvature thereof and the shank portion depends on the shape of the head portion and cannot be varied any more after the head portion has been manufactured. In particular, the lateral distance of the humerus from the joint cannot be adjusted.

US-A-3 102 536 discloses an endoprosthesis for a hip joint which endoprosthesis comprises an elongate shank portion having a distal and a proximal portion, the proximal portion having an end portion which is inclined with respect to the axis of the shank portion, the endoprosthesis further comprising a separate head portion having a spherical joint surface. The neck portion of the shank portion or stem is cylindrical and adapted to be accommodated by a blind bore or a ball head. The wall of the blind bore has peripherally spaced grooves extending parallel to the axis of the bore and having different lengths. A radially extending pin on the neck portion is adapted to engage one of the grooves in order to vary the position of the ball head on the neck portion. The ball head is fastened to the neck portion by a screw extending through a hole coaxial to the blind bore of the ball portion and adapted to be threaded into a threaded bore at the proximal end of the neck portion. In another embodiment of the known hip joint, the neck portion has an outer thread to be threaded into the threaded blind bore of the ball head.

It is an object of the present invention to provide an endoprosthesis for a shoulder joint that provides for an adjustable distance between the head portion and the shank portion.

According to the invention the head portion of the endoprosthesis is connected to the proximal end of the shank portion through a threaded connection such that the distance between the head portion and the shank portion can be varied. The head portion is screwed into the shank portion until the desired distance is obtained.

The threaded portion is formed as a threaded pin having an outer thread and the second threaded portion is formed by a threaded blind bore. On the one side this facilitates the handling when the head portion is screwed in and, on the other side, the head portion can be secured against rotation in the shank portion.

The threaded pin includes peripherally spaced longitudinal grooves extending parallel to the axis, the grooves preferably including a rounded transverse profile to secure the pin against a rotation.

A locking pin is turned into a threaded bore extending transversally with respect to the axis of the blind bore. This locking pin can engage a groove of the threaded pin to secure the pin against rotation. The locking pin preferably includes a rounded tip and a head formed to be engaged by a turning tool. According to a preferred embodiment of the invention, the shaft of the locking screw includes a transversally extending plastic insert. The plastic insert prevents an accidental release of the rotary lock.

The center of curvature of the joint surface is located on the axis of the threaded pin. This allows to symmetrically form the head portion which has advantages when being screwed into the blind bore.

According to an embodiment of the invention, the axis of the second threaded portion is inclined with respect to the longitudinal axis of the shank portion. This makes it possible to adjust the lateral distance between the joint and the humerus. The angle of inclination is preferably about 50° to conform to the natural joint.

According to a further embodiment of the invention, the head portion includes reference markings provided at the edge or the lower side of the joint surface which markings are aligned to the grooves. Thus, the surgeon can observe the orientation of the grooves even when the thread is not visible.

In a further embodiment of the invention, the head portion includes recesses to be engaged by a turning tool to exert a torque which recesses are located close to the edge or at the lower side of the joint surface.

Still further, the grooves are spaced about 90°, and the respective markings are formed by the recesses.

The shank portion includes a proximal portion which may have openings, preferably axially extending elongate openings and a smooth distal portion. The distal shank portion entering the humerus may comprise a smooth surface, while the openings in the proximal shank portion facilitate an anchoring by means of bony material growing in.

The shank portion in close proximity to the proximal end of the blind bore may include a collar extending perpendicularly with respect thereto, and the shank portion includes an oval cross section in the range of the distal end of the blind bore. The contact areas between bone, shank and head may be thus designed to have the appropriate shape.

In a further embodiment of the invention, the head portion, the shank portion and/or the locking screw are made of metal, preferably titan or a cobalt-chrome alloy.

Embodiments of the present invention are now being described with reference to the accompanying drawings.
- Fig. 1: shows a side view, partly in section, of an endoprosthesis for a shoulder joint according to the invention.
- Fig. 2: shows a side view of the head portion of the endoprosthesis of Fig. 1.
- Fig. 3: shows a top view of the head portion according to Fig. 2 from the distal end.
- Fig. 4: shows a side view, partly in section, of a shank portion according to the invention.
- Fig. 5: shows a front view of the shank portion of Fig. 4.
- Fig. 6: shows a cross-sectional view of the shank portion of Fig. 4 taken along line A-A.

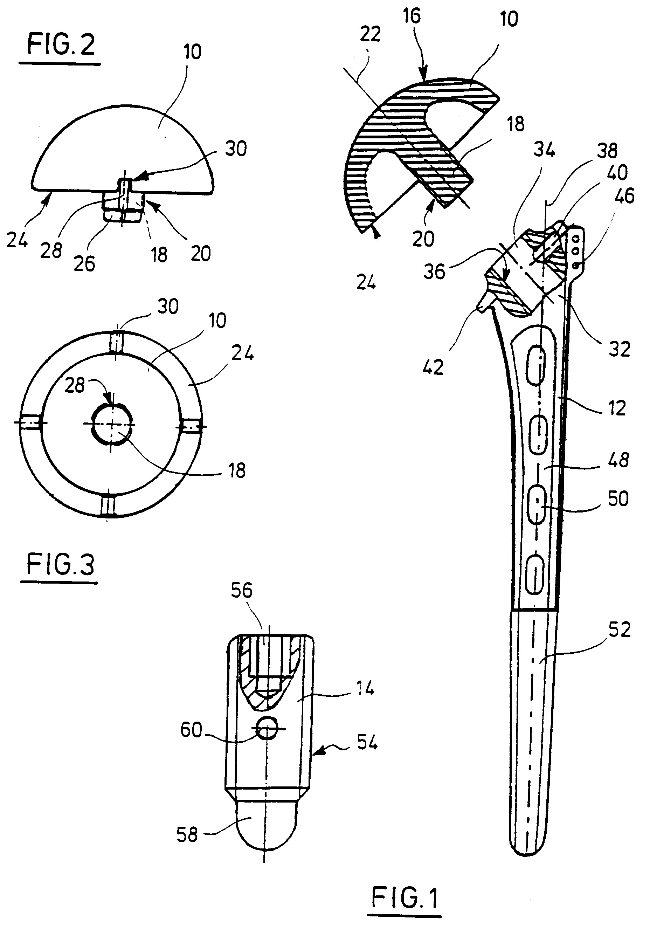

With reference to Fig. 1, the endoprosthesis for a shoulder joint comprises a head portion 10 which is screwed into a shank portion 12 and which is secured by a locking pin 14. The head portion 10 formed as a rotating head may cooperate with an artificial joint socket (not shown) in a natural shoulder bone (likewise not shown) to define a shoulder joint.

The head portion 10 includes a spherically shaped joint surface 16 and a threaded pin 18 including an outer thread 20. The joint surface 16 is approximately semi-spherically shaped with the center of curvature being located on the axis 22 of the threaded pin. The joint surface terminates in an edge 24. As Fig. 2 and 3 show in some detail the outer side 20 of the threaded pin 18 extending up to a recess 26 provided at the free end of the pin.

The threaded pin 18 includes four peripherally spaced grooves extending parallel to the axis 22 and having a rounded cross-sectional profile. Four markings shaped as engaging recesses 30 are provided along the edge 24 of the joint surface 16 which markings are aligned with longitudinal grooves 28 of the threaded pin 18.

The proximal end 32 of the shank portion 12 includes a threaded portion 36 in a blind bore 34 cooperating with the outer thread 20 of the pin. The axis 22 is inclined at an angle with respect to the longitudinal axis 38 of the shank portion 12. The proximal end 32 of the shank portion 12 further includes a threaded bore 40 extending perpendicular with respect to the axis 22 for receiving the locking pin 14, said shank portion further comprising a peripheral collar 42 likewise extending perpendicularly with respect to the axis 22. Still further a peen having three openings 46 is provided close to the proximal end of the shank portion.

The shank portion 12 has a proximal portion 48 including axially extending elongate openings 50 and a smooth distal portion 52.

The locking pin 14 includes an outer thread 54, a head 56 shaped to be engaged by a turning tool, and a rounded tip 58. The pin 14 further includes a transverse bore 60 accommodating a plastic insert.

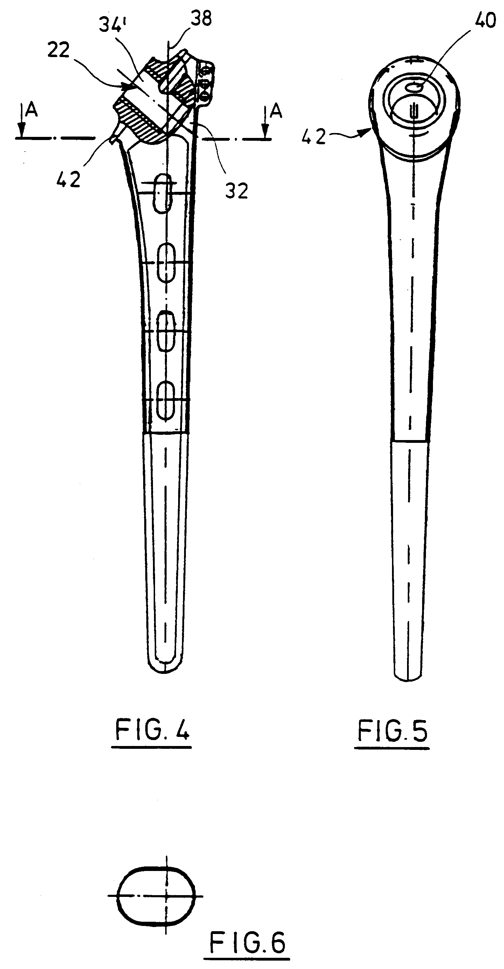

Figs. 4 to 6 show an alternative embodiment in further details. The threaded bore 34' cooperating with a threaded pin of a head portion is shaped as a blind bore. All the remaining features shown correspond to the embodiment described with reference to Fig. 1.

The concentrical shape of the collar 42 with respect to the axis 22 is clearly visible. Fig. 6 shows the oval cross section of the shank with respect to axis 38 in the section A-A in the range of the distal end 32.

In operation, the shank portion 12 with the smooth distal portion 52 being directed forwardly is driven into the humerus. The head portion 10 is guided along the axis 22 towards the shank portion 12, and is screwed with the threaded pin 18 into the threaded bore 34 or 34' until the desired distance between the joint surface 16 and the shank portion 12 with respect to its axis 38, for example, is obtained. The next mark or, respectively recess 30 will be aligned with the bore 34, 34' and the locking pin 14 including the plastic insert is fixed in the threaded bore 40. The pin-type screw 14 is tightened until its rounded tip 58 engages a groove 28 of pin 18. The head 10 is now locked against rotation about axis 22, while the plastic insert prevents a loosening locking pin 14. Now the endoprosthesis including the joint surface 16 may be inserted into the bone (not shown) and fractured parts, if any, may be secured to the peen. The insertion of the prosthesis 13 free of cement and bony material may grow into the elongate openings 50 of the proximal shank portion 48.

## Claims

1. An endoprosthesis for a shoulder joint, comprising an elongate shank portion (12) adapted to be driven into the proximal humerus and having a distal portion (52) and a proximal portion (48), the proximal portion (48) having an end portion (32) which is inclined with respect to the axis of the shank portion (12) and having a blind bore (34, 34') and being provided with an internal thread (36), a separate head portion (10) having a spherical joint surface (16) and a pin portion (18) opposite to the joint surface (16), the pin portion (18) having an external thread (20) and being adapted to be threaded into the threaded blind bore (34, 34'), the pin portion (18) further having peripherally spaced grooves (28) extending parallel to the axis of said pin portion (18), the end portion (32) having a transverse threaded bore (40) which accommodates a threaded locking pin (14) adapted to engage one of the grooves (28) to secure said pin portion (18) against rotation.

2. The endoprosthesis of claim 1, wherein the axis of the proximal end portion (32) is inclined with respect to the axis of said shank portion (12) at an angle of essentially 50°.

3. The endoprosthesis of claim 1 or 2, wherein the longitudinal grooves (28) have a rounded cross-sectional profile.

4. The endoprosthesis of one of the claims 1 to 3, wherein the head portion (10) includes reference marks which are provided at or close to the edge or at the lower side of the joint surface (16), the reference markings being aligned to the longitudinal grooves (28).

5. The endoprosthesis of one of the claims 1 to 4, wherein the head portion (10) includes recesses (30) for the engagement by a turning tool, the recesses (30) being provided at or close to the edge or at the lower side of the joint surface (16).

6. The endoprosthesis of claims 4 and 5, wherein the markings are defined by the recesses (30).

7. The endoprosthesis of one of the claims 1 to 6, wherein four longitudinal grooves (28) are provided peripherally spaced at 90°.

8. The endoprosthesis of one of the claims 1 to 7, wherein the locking pin (14) includes a rounded tip (58).

9. The endoprosthesis of one of the claims 1 to 8, wherein the locking pin (14) includes a head which is adapted to be engaged by a turning tool.

10. The endoprosthesis of one of the claims 1 to 9, wherein the shank (12) of the locking pin (14) includes a transversally extending plastic insert (60).

11. The endoprosthesis of one of the claims 1 to 10, wherein the head portion (10), the shank portion (12) and/or the locking pin (14) are formed of metal.

12. The endoprosthesis of claim 11, wherein the shank portion (12) and/or the locking screw (14) are formed of titan or a cobalt-chrome alloy.

## Patentansprüche

1. Endoprothese für das Schultergelenk mit einem länglichen, in den proximalen Humerus einschlagbaren Schaftteil (12), das einen distalen Abschnitt (52) und einen proximalen Abschnitt (48) aufweist, wobei der proximale Abschnitt (48) einen Endabschnitt (32) hat, der gegen die Längsachse (38) des Schaftteils (12) geneigt ist und eine Sackbohrung (34, 34') aufweist mit einem Innengewinde (36), einem Kopfteil (10), das eine sphärische Gelenkläche (16) und der Gelenkfläche (16) gegenüberliegend einen Zapfen (18) mit Außengewinde (20) aufweist, der in das Gewinde der Sackbohrung (34, 34') einschraubbar ist, wobei der Zapfen (18) ferner achsparallele, in Umfangsrichtung beabstandete Längsnuten (28) aufweist und der Endabschnitt (32) eine Gewindebohrung (40) aufweist, die eine Sicherungsschraube (14) aufnimmt, die mit einer der Nuten (28) zusammenwirkt, um den Zapfen (18) gegenüber Drehung zu sichern.

2. Endoprothese nach Anspruch 1, bei der die Achse des proximalen Endabschnitts (32) gegenüber der Achse des Schaffteils (12) um einen Winkel von im wesentlichen 50° geneigt ist.

3. Endoprothese nach Anspruch 1 oder 2, bei der die Längsnuten (28) ein gerundetes Querschnittsprofil aufweisen.

4. Endoprothese nach einem der Ansprüche 1 bis 3, bei der das Kopfteil (10) Markierungen aufweist, die nahe dem Rand bzw. an der Unterseite der Gelenkfläche (16) vorgesehen sind und die zu den Längsnuten (28) orientiert sind.

5. Endoprothese nach einem der Ansprüche 1 bis 4, bei der das Kopfteil (10) Ausnehmungen (30) für den Eingriff eines Drehwerkzeugs aufweisen, wobei die Ausnehmungen (30) an oder nahe dem Rand bzw. an der Unterfläche der Gelenkfläche (16) vorgesehen sind.

6. Endoprothese nach den Ansprüchen 4 und 6, bei der die Markierungen durch die Ausnehmungen (30) gebildet sind.

7. Endoprothese nach einem der Ansprüche 1 bis 6, bei der vier Längsnuten (28) im Umfangsabstand von 90° vorgesehen sind.

8. Endoprothese nach einem der Ansprüche 1 bis 7, bei der die Sicherungsschraube (14) eine gerundete Spitze (58) aufweist.

9. Endoprothese nach einem der Ansprüche 1 bis 8, bei der die Sicherungsschraube (14) einen Kopf aufweist, der für den Eingriff mit einem Drehwerkzeug geformt ist.

10. Endoprothese nach einem der Ansprüche 1 bis 9, bei der der Schaft der Sicherungsschraube (14) einen sich quer erstreckenden Einsatz (60) aus Kunststoff aufweist.

11. Endoprothese nach einem der Ansprüche 1 bis 10, bei der das Kopfteil (10), das Schaftteil (20) und/oder die Sicherungsschraube (14) aus Metall geformt sind.

12. Endoprothese nach Anspruch 11, bei der das Schaftteil (12) und/oder die Sicherungsschraube (14) aus Titan oder einer Kobalt-Chrom-Legierung geformt sind.

## Revendications

1. Endoprothèse d'une articulation de l'épaule, comprenant une partie allongée de tige (12) destinée à être implantée dans l'humérus proximal et ayant une partie distale (52) et une partie proximale (48), la partie proximale (48) ayant une partie d'extrémité (32) qui est inclinée sur l'axe de la partie de tige (12) et qui comporte un trou borgne (34, 34') et comportant un taraudage (36), une partie séparée de tête (10) ayant une surface sphérique d'articulation (16) et une partie de broche (18) située sur le côté opposé de celui de la surface d'articulation (16), la partie de broche (18) comportant un filetage (20) et étant destinée à être vissée dans le trou borgne taraudé (34, 34'), la partie de broche (18) comportant par ailleurs des rainures (28) distantes les unes des autres à la périphérie et parallèles à l'axe de ladite partie de broche (18), la partie d'extrémité (32) comportant un trou taraudé transversal (40) qui loge une cheville filetée de blocage (14) destinée à pénétrer dans l'une des rainures (28) pour immobiliser ladite partie de broche (18) de manière à l'empêcher de tourner.

2. Endoprothèse selon la revendication 1, dans laquelle l'axe de la partie proximale d'extrémité (32) est incliné sur l'axe de ladite partie de tige (12) suivant un angle essentiellement de 50°.

3. Endoprothèse selon la revendication 1 ou 2, dans laquelle les rainures longitudinales (28) ont un profil arrondi en coupe transversale.

4. Endoprothèse selon l'une des revendications 1 à 3, dans laquelle la partie de tête (10) comporte des repères de référence qui sont réalisés sur ou à proximité du bord ou sur le côté inférieur de la surface d'articulation (16), les repères de référence étant alignés sur les rainures longitudinales (28).

5. Endoprothèse selon l'une des revendications 1 à 4, dans laquelle la partie de tête (10) comporte des cavités (30) dans lesquelles un outil destiné à la faire tourner peut pénétrer, les cavités (30) étant réalisées sur ou à proximité du bord ou sur le côté inférieur de la surface d'articulation (16).

6. Endoprothèse selon les revendications 4 et 5, dans laquelle les repères sont formés des cavités (30).

7. Endoprothèse selon l'une des revendications 1 à 6, dans laquelle quatre rainures longitudinales (28) sont réalisées à la périphérie et distantes les unes des autres de 90°.

8. Endoprothèse selon l'une des revendications 1 à 7, dans laquelle la cheville de blocage (14) comporte un bout arrondi (58).

9. Endoprothèse selon l'une des revendications 1 à 8, dans laquelle la cheville de blocage (14) comporte une tête adaptée à être engagée par un outil d'entraînement en rotation.

10. Endoprothèse selon l'une des revendications 1 à 9, dans laquelle la tige de la cheville de blocage (14) comporte une pièce insérée en matière plastique (60) s'étendant transversalement.

11. Endoprothèse selon l'une des revendications 1 à 10, dans laquelle la partie de tête (10), la partie de tige (12) et/ou la cheville de blocage (14) sont réalisées en métal.

12. Endoprothèse selon la revendication 11, dans laquelle la partie de tige (12) et/ou la vis de blocage (14) sont réalisées en titane ou en alliage de cobalt et de chrome.
